## Europäisches Patentamt
## European Patent Office
## Office européen des brevets

(19)

(11) Publication number: **0 102 070**
**A2**

(12)

# EUROPEAN PATENT APPLICATION

(21) Application number: **83108433.0**

(22) Date of filing: **26.08.83**

(51) Int. Cl.³: **A 61 B 5/14**

(30) Priority: **27.08.82 DK 3854/82**

(43) Date of publication of application:
**07.03.84 Bulletin 84/10**

(84) Designated Contracting States:
**AT BE CH DE FR GB IT LI LU NL SE**

(71) Applicant: **RADIOMETER A/S**
**Emdrupvej 72**
**DK-2400 Copenhagen NV(DK)**

(72) Inventor: **Andersen, Jorgen**
**Dyrespringvej 3**
**DK-2730 Herlev(DK)**

(74) Representative: **Patentanwälte Grünecker, Dr.**
**Kinkeldey, Dr. Stockmair, Dr. Schumann, Jakob, Dr.**
**Bezold, Meister, Hilgers, Dr. Meyer-Plath**
**Maximilianstrasse 58**
**D-8000 München 22(DE)**

(54) **A liquid sampler.**

(57) A liquid sampler which may be used for collecting samples of arterial blood is preferably in the form of a syringe. A venting passage (25, 31) is defined in the syringe piston (12) so that gas may escape through the venting passage when arterial blood flows into the cylinder space (17) under the influence of the arterial blood pressure. The venting passage is controlled by valve means (19, 21), which automatically close the venting passage when the cylinder space has been filled with blood and the blood starts flowing into the venting passage. The venting passage is closed by liquid resistant valve surface parts, and movement of these surface parts to their closing position is caused by a liquid reacting substance (22) which is exposed to the liquid sample flowing into the venting passage. This liquid reacting substance may, for example, be a material which swells, expands, or is softened when contacted by liquid. Alternatively, the liquid reacting substance may be a liquid soluble material.

Fig. 4

PATENTANWÄLTE
GRÜNECKER · KINKELDEY DR. STOCKMAIR
DR. SCHUMANN · JAKOB DR. BEZOLD · MEISTER
HILGERS · DR. MEYER · PLATH

# A LIQUID SAMPLER

The present invention relates to a liquid sampler of the type which may be used for collecting a liquid sample substantially without contamination by gases from the ambient atmosphere. While the liquid sampler according to the invention may be used for collecting samples from liquids of any type, the sampler is especially suited for collecting a blood sample, such as an arterial blood sample, from a blood vessel for subsequent blood gas analysis.

The blood parameters determined by blood gas analysis, such as the partial pressure of oxygen ($pO_2$), the partial pressure of carbon dioxide ($pCO_2$), and the acidity (pH), may be influenced and changed when the blood sample comes into contact with the ambient atmosphere, and, consequently, it is necessary to take special measures in order to avoid or reduce such contact.

Blood samplers in the form of a syringe, wherein the blood collecting chamber is vented to the atmosphere through a venting passage while a blood sample is collected, and wherein the venting passage may be closed when a suitable amount of blood has been collected in the syringe, are well known, for example from US patents Nos. 3,943,917, 4,133,304, 4,206,768 and 4,257,426 and German Offenlegungsschrift No. 3,041,563. However, all of these known blood collecting syringes require certain manual operations subsequent to the collection of a blood sample in order to close the venting passage.

US patents Nos. 3,960,139, 3,978,846, 4,266,558, 4,266,559, 4,327,745, 4,340,067, and 4,373,535, PCT publication No. WO 81/03426, and published European patent applications Nos. 47,176 and 47,806 all disclose blood samplers comprising a collecting chamber, which is vented to the atmosphere through a filter or closure element, which is pervious to gas, but impervious to liquid or becomes impervious to liquid when wetted thereby. This latter structure causes the flow of blood sample into the blood collecting chamber of the sampler to be automatically stopped, when the chamber has been filled so that blood comes into contact with the filter or closure element. The prior art also comprises a syringe disclosed in US patent No. 4,299,238.

Although the filter or closure elements described in the above mentioned publications are able to stop the flow of the blood sample, an undesirably high gas exchange between the collected blood sample and the ambient atmosphere may still take place through the filter element, even when the filter or closing element is made from a material which swells or reacts in some other respect when contacted by liquid.

US patent No. 4,361,155 discloses a blood sampling syringe, wherein a venting passage formed in the syringe piston or plunger may be closed by means of a floating stopper arranged within the piston cylinder. However, in this known structure the closure efficiency of the stopper when the syringe has been filled with the blood is dependent on the orientation of the syringe.

The present invention provides a liquid sampler, wherein a venting passage of a sample container is automatically closed when the sample container is filled with a liquid sample, so as to form a reliable, gas tight seal, which is not affected by the orientation of the sampler.

Thus, the present invention provides a liquid sampler comprising a sample container for receiving a liquid sample in the inner space thereof and having a sample inlet passage and a venting passage each communicating with the inner space of the sample container, a liquid reacting substance arranged so as to become exposed to liquid flowing into the venting passage, and the sampler according to the invention is characterized in that the venting passage is controlled by valve means defining valve surface parts, which are made from a liquid resistant material and at least partly define the venting passage and which are mutually movable from an open position to a closed position in which the venting passage is closed, and in that the liquid reacting substance is adapted to cause said valve surface parts to move from their open to their closed position when said substance has been exposed to and has reacted with liquid.

P&V F3563 jB, KEV/IPb, 1983 08 23

In the sampler according to the invention the venting passage is closed by the valve surface parts, which are moved to their closed position when the inner space of the sample container has been filled or substantially filled with a liquid sample. As these valve surface parts are made from a liquid resistant material, for example plastic, rubber, glas and/or metal, an efficient and a reliable sealing of the venting passage may be obtained. The liquid reacting substance may be of any type which is able to cause movement of the valve surface parts to their closed position, when the liquid reacting substance is contacted by the liquid sample.

As an example, the liquid reacting substance may comprise a substance which expands when contacted by liquid so as to move said valve surface parts into mutually sealing engagement. In that case the liquid reacting substance serves as driving means for moving the valve surface parts to their closed position. Thus, the liquid reacting substance may comprise a body including fibrous material of a type which swells when contacted by liquid.

The valve surface parts may have any suitable form provided that they are able to close the venting passage. Thus, for example, the valve surface parts may be formed by inner surface parts of a compressible tube, which is pinched or compressed when the liquid reacting substance is exposed to liquid and caused to expand.

According to another embodiment the valve means comprise a movable valve body and a valve seat, and the liquid reacting substance may then be arranged so as to move the valve body into sealing engagement with the valve seat when expanding. As an example, the liquid reacting substance may be arranged within a chamber which is closed by a displaceable piston or wall which is connected to the valve body.

The liquid reacting substance may be arranged in the venting passage upstream of or at the valve means. In the preferred embodiment, however, the liquid reacting substance is arranged downstream of the valve means, whereby it may be ensured that all gas or air has been vented from the inner space of the sample container when the valve surface parts are moved to their closed position, and that liquid, which has been in contact with air or gas during the sampling procedure has passed the valve surface parts when they are moved to their closed position.

The liquid reacting substance may be arranged within a space which communicates with the venting passage. Alternatively, the liquid reacting substance may be located within the venting passage itself in an enlarged section thereof, and in that case the valve body may be arranged at the inlet end of the enlarged section, said inlet end forming the valve seat. The liquid reacting substance allows air and gas flowing through the venting passage to pass. However, when the inner space of the sample container has been filled with liquid, and the liquid flows into the venting passage to the enlarged section thereof, the liquid reacting substance expands and presses the valve body into sealing engagement with the valve seat.

In a simple embodiment the valve body is in the form of a disc-like member arranged between the liquid reacting substance and the inlet end of the enlarged section. The disc-like member may then be shaped or arranged so as to be out of sealing engagement with the valve seat, until the disc-like member is biased towards the valve seat by the expanding liquid reacting substance. In a preferred embodiment the disc-like member is hinge-connected so as to be swingable from an open to a closed, valve seat engaging position.

According to a further embodiment the liquid sampler may comprise valve surface parts biased towards their closed position, the liquid reacting substance comprising a material, which in its dry condition is rigid and retains the valve surface parts in their open position, and which becomes unable to counteract the bias of the valve surface parts when contacted by liquid, whereby the valve surface parts are moved to their closed position when the liquid reacting substance comes into contact with liquid. The dry, rigid liquid reacting substance may be able to resist compressive and/or tensile forces so as to counteract the bias of the valve surface parts. When the liquid reacting substance is able to resist tensile forces it may, for example, be in the form of strings retaining the valve surface parts in their open position. When the liquid reacting substance is able to resist compressive forces it may, for example, be in the form of spacing means positioned between the valve surface parts so as to keep the surface parts separated in their open position.

The liquid reacting substance may, for example, be a liquid soluble material, such as crystals of a mineral salt, which are dissolved when contacted by a liquid.

According to an alternative embodiment, the liquid sampler may further comprise biasing means for biasing the valve surface parts towards their closed position, the liquid reacting substance being liquid softenable, whereby the valve surface parts are moved to their closed position by the biasing means when the liquid reacting substance comes into contact with liquid. The liquid softenable material, which is rigid in its dry condition, may, for example, be porous so as to allow gas and air to pass therethrough. When, however, the softenable material is contacted by liquid it may obtain a plastic condition, whereby it is compressed by the biasing means and may form a gas tight sealing substance located between the valve surface parts.

When the liquid sampler according to the invention is used for collecting blood from a donor's blood vessel, the sampler is preferably in the form of a syringe, and the venting passage may then be defined in the piston.

The invention will now be further described with reference to the drawings, wherein

Fig. 1 is a side view and partially sectional view of an embodiment of the liquid sampler according to the invention in the form of a syringe for collecting blood samples from a blood vessel of a donor,

Fig. 2 is a perspective view of the piston or plunger of the syringe shown in Fig. 1,

Fig. 3 is an exploded perspective view of the piston or plunger shown in Fig. 2,

Fig. 4 is a sectional view of the left end portion of the syringe of Fig. 1 shown in an enlarged scale,

Fig. 5 is a cross-sectional view along the line 5-5 in Fig. 4,

Fig. 6 is a cross-sectional view along the line 6-6 in Fig. 4,

Figs. 7A, 7B, and 7C illustrate alternative embodiments of the front end of the piston or plunger shown in a cross-sectional view along the line 7-7 in Fig. 4,

Figs. 8A, 8B, and 8C are plan views of different embodiments of a valve member or closure member for use in the piston or plunger of the syringe shown in Fig. 4,

Figs. 9A, 9B, and 9C are side views of the valve member or closure member shown in Figs. 8A, 8B, and 8C, respectively,

Fig. 10 is a side view and partially sectional view of a second embodiment of the liquid sampler according to the invention also in the form of a syringe for collecting blood samples,

Fig. 11 is a sectional view of the left hand end portion of the syringe of Fig. 10 shown in an enlarged scale,

Fig. 12 is a cross-sectional view along the line 12-12 in Fig. 11,

Fig. 13 is a sectional view of the inner or left hand end of the piston or plunger of the syringe shown in Figs. 10-12, and illustrating an alternative embodiment of the valve means for closing the venting passage defined in the piston or plunger,

Fig. 14 is a cross-sectional view along the line 14-14 in Fig. 13,

Fig. 15 is a sectional view of the inner or left hand end of the piston or plunger of the syringe shown in Figs. 10-12, and illustrating a further embodiment of the valve means for closing the venting passage,

Fig. 16 is a cross-sectional view along the line 16-16 in Fig. 15, and

Fig. 17 is a perspective view of a clamping device forming part of the valve means shown in Figs. 15 and 16.

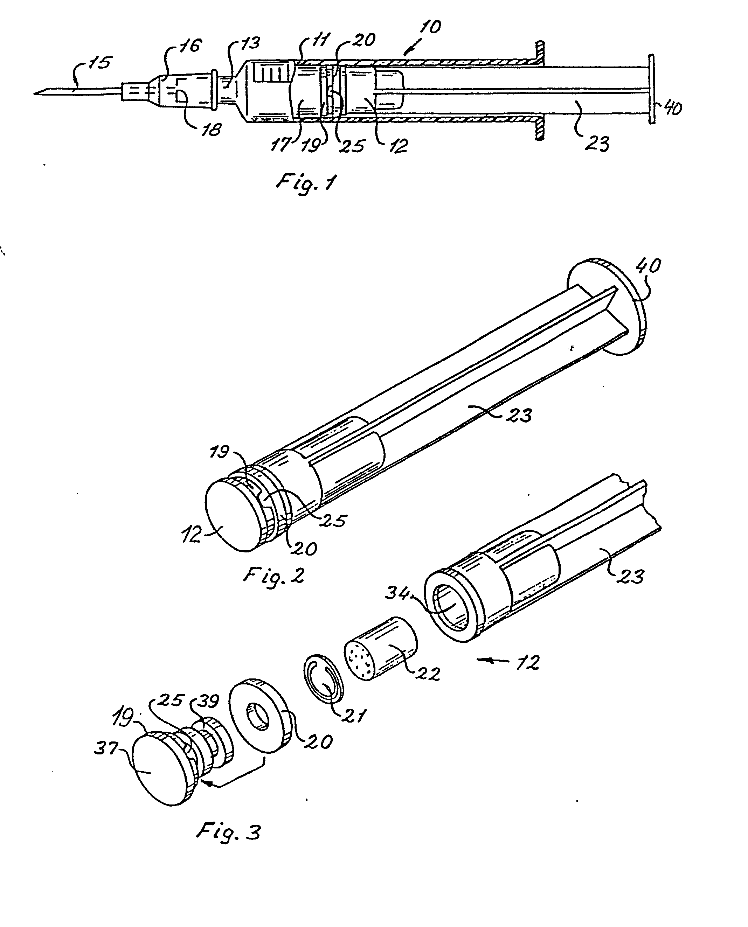

Figs. 1-6 illustrate an embodiment of the liquid sampler according to the invention in the form of a syringe 10. The syringe 10 comprises a syringe cylinder or barrel 11 and a piston or plunger 12, which comprises a plurality of separate elements. The syringe cylinder 11 may, for example, be made from glass or plastic, and may be provided with a suitable gas impervious barrier layer, not shown. At its front end, the syringe cylinder 11 is provided with a hollow neck 13 for mounting a hollow needle 15 thereon. The outer peripheral surface of the hollow neck has a frusto-conical shape and is adapted to cooperate with a complementary inner frusto-conical surface of a mounting socket 16 arranged at one end of the hollow needle 15.

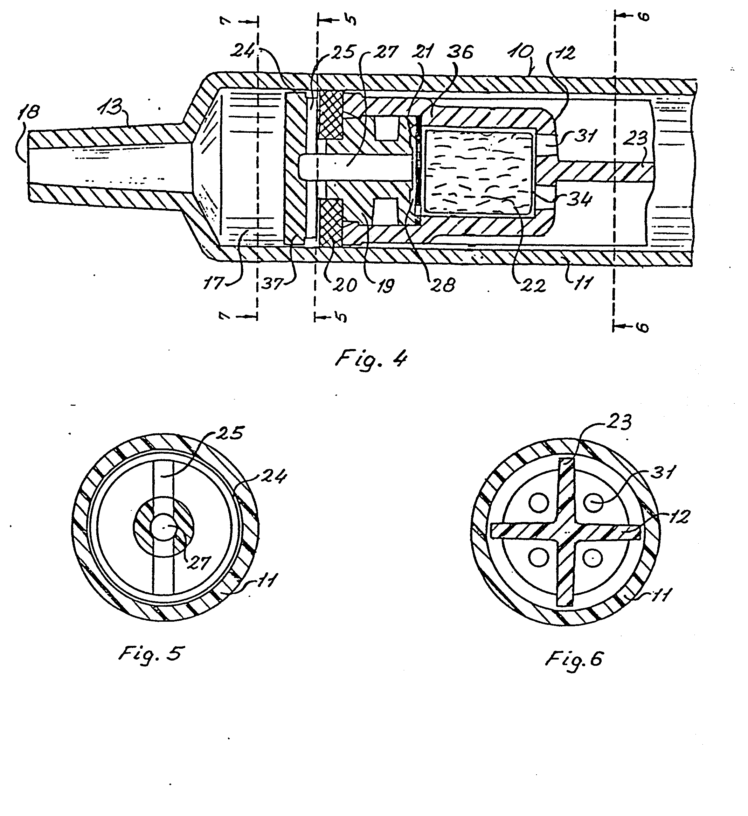

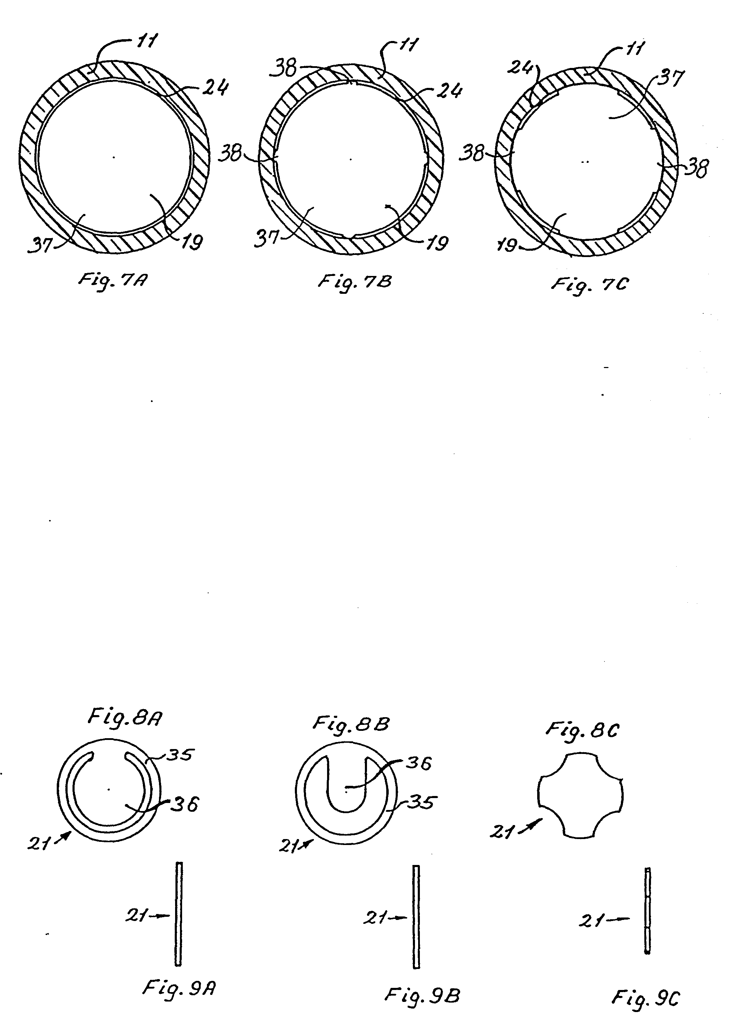

The syringe cylinder 11 and the piston or plunger 12 displaceably arranged therein define a sample collecting chamber 17. When the hollow needle 15 is mounted on the neck 13 the inner bore of the needle 15 is in communication with the sample collecting chamber 17 through an inlet bore 18 extending axially through the neck 13 of the syringe cylinder 11. As shown in Fig. 3, the piston or plunger 12 comprises a plurality of separate parts, namely a front end element 19, a sealing ring 20, a valve member or closure member 21, an expandable body made from a liquid reacting material 22, and a piston rod 23. The front end element 19 of the piston defines an inner space 27. Figs. 4 and 5 which is in communication with the sample collecting chamber 17 through one or more connecting passages 25. At its inner end the front end element 19 comprises a cylindrical part 37 having an outer diameter, which is slightly smaller than that of the syringe cylinder 11 so as to define a narrow, annular passage 24 between the inner wall of the syringe cylinder and the outer peripheral surface of the part 37, vide Fig. 7A. Figs. 7B and 7C illustrate alternative embodiments where the cylindrical part 37 is provided with axially extending outer ridges or ribs 38, which in Fig. 7B have a more narrow peripheral width than in Fig. 7C. The connecting passages 25 extend substantially radially between the hollow space 27 and the annular passage 24 and are preferably substantially uniformly spaced along the periphery of the cylindrical part 37 of the piston element 19. The sealing ring 20 is received in an annular channel 39, Fig. 3, which is formed in the piston element 19 adjacent to, but behind the radial connecting passages 25. The outer end of the piston element 19 is fastened to the piston rod 23 as best shown in Fig. 4.

The shape and size of the sealing ring 20 and the material from which it is made are chosen and adapted to the size of the material of the syringe cylinder 11 so as to obtain sealing engagement between the

P&V F3563 jB, KEV/IPb, 1983 08 23

sealing ring and the inner surface of the cylinder, and so as to obtain friction which is sufficiently low to ensure that the piston may easily be moved manually in relation to the cylinder. An open hollow space 34 defined within the front end of the piston rod 23 is adapted to receive the adjacent end portion of the piston element 19, the valve member or closure member 21, and the expandable body 22. The hollow space 34 is in communication with the open end of the cylinder 11 and consequently with the ambient atmosphere through connecting passages 31. A disc-like member 40, Fig. 2, formed at the outer end of the piston rod 23 serves as a gripping portion.

The piston element 19, the sealing ring 20, the valve member 21, the expandable body 22, and the piston rod 23 cooperate so as to form valve means which are initially in an open position in which gas or air may flow freely from the sample collecting chamber 17 to the ambient atmosphere, and which are automatically moved to and retained in a closed position when blood or another aqueous liquid penetrates into the hollow space 34 and comes into contact with the expandable body 22. In Fig. 4 the valve means are shown in their open position, as the expandable body 22 has not yet been contacted by liquid. In this condition the sample collecting chamber 17 is in communication with the ambient atmosphere through a venting passage formed by the annular passage 24, the connecting passages 25, the hollow space 27, a space 28 defined between the piston element 19 and the valve member 21, the hollow space 34, and the connecting passages 31.

The valve member or closure member 21 which is shown more in detail in Figs. 8A and 9A is arranged between the expandable body 22 and the adjacent end surface of the piston element 19 and comprises an annular frame part 35 and a closure element 36 hinge-connected thereto. Figs. 8B and 9B show an embodiment of the closure member 21 with a differently shaped closure element 36, and Figs. 8C and 9C show an embodiment of the closure member 27 adapted to be loosely arranged between the piston element 19 and the body 22. In the latter case the valve member or closure member 21 has a size and shape so as to extend across the adjacent opening of the hollow space 27 regardless of orientation of the syringe.

The syringe described above with reference to Figs. 1-9 may be used for collecting an arterial blood sample. The syringe cylinder 11 may then be held by one hand, and by means of the other hand the piston 12 may be moved to a position in which the volume of the sample collecting chamber 17 corresponds to the desired blood sample volume. A hollow needle 15 of a well known type is then mounted on the neck of the syringe cylinder, and the pointed free end of the needle is inserted into an artery. When the needle has penetrated into the artery the blood pressure therein causes arterial blood to flow through the hollow needle and into the collecting chamber 17 defined within the syringe cylinder 11. As the arterial flows into the chamber 17, air or gas is expelled therefrom and may escape through the venting passage mentioned above, namely through the annular passage 24, the connecting passages 25, the hollow space 27, the space 28, past the disc-like valve member or closure member 21 into the hollow space 34, and out into the ambient atmosphere through the connecting passages 31.

When the collecting chamber 17 has been completely filled by arterial blood and the air or gas has substantially or completely been expelled from the collecting chamber 17, the blood pressure within the artery causes some of the blood from the collecting chamber 17 to flow through the annular passage 24, the connecting passages 25, the hollow space 27, the space 28, and to penetrate into the hollow space 34 where it comes into contact with the expandable body 22 which is made from the liquid reacting material of a type, which expands when contacted by liquid. The dimensions of the hollow space 34 and of the expandable body 22 received therein are selected so that the pressure exerted on the closure member 21 by the expanding body 22 exceeds the pressure within the blood collecting chamber 17 which is in communication with the artery. Consequently, the closure member or valve member 21 is pressed into sealing engagement with a valve seat formed at the adjacent end of the piston element 19, whereby the venting passage is closed so that further flow of blood into and out from the blood collecting chamber 17 is stopped and contamination of the collected sample with gas from the ambient atmosphere is effectively prevented, whereby more reliable results may be obtained in any subsequent blood gas analysis. When the valve means have been closed automatically as described above, the hollow needle 15 may be removed from the artery and thereafter the hollow needle may also be removed from the syringe. The collected blood sample may now be injected directly into a blood gas analysing apparatus.

The expandable body 22 may be made from a material of any type which is able to press the valve member or closure member 21 into sealing engagement with its seat when the material is contacted by blood or another aqueous liquid. Such materials may, for example, comprise fibrous materials such as cellulose fibres, for example of the type used in babies' napkins, textile fibres, such as viscose rayon, wool, cotton, and jute. The expandable body may also comprise materials or substances, which expand and are formed into a gel when the material or substance adsorbs an aqueous liquid. Such materials or substances comprise starch-based polymer materials of the type used in babies' napkins and in bandages, and freeze-dried, compressed products.

In the syringe shown in Figs. 1-9, the valve member or closure member 21 has a function in addition to that described above. Thus, the valve member functions as a one-way valve when the piston or plunger 12 is moved in the syringe cylinder 11 in order to increase or reduce the volume of the sample collecting chamber 17. When the piston is moved outwardly so as to increase the volume of the chamber 17, the closure member 21 is automatically moved to its closed position, while the closure member is moved to its open position when the piston is moved in the opposite direction in order to reduce the volume of the chamber 17. Therefore, if the arterial blood pressure of a patient from which a blood sample is collected is insufficient to cause blood to flow into the syringe cylinder, the piston 12 may be moved to its innermost position adjacent to the neck 13 before the hollow needle is inserted into the artery, and during the forward movement of the piston air may escape from the sample collecting chamber 17 through the open venting passage defined in the piston. The pointed end of the hollow needle is now inserted into the artery, and a blood sample may be retracted therefrom by slowly moving the piston or plunger 12 outwardly so as to increase the volume of the sample collecting chamber 17 and create an underpressure therein whereby arterial blood is sucked from the artery into the syringe.

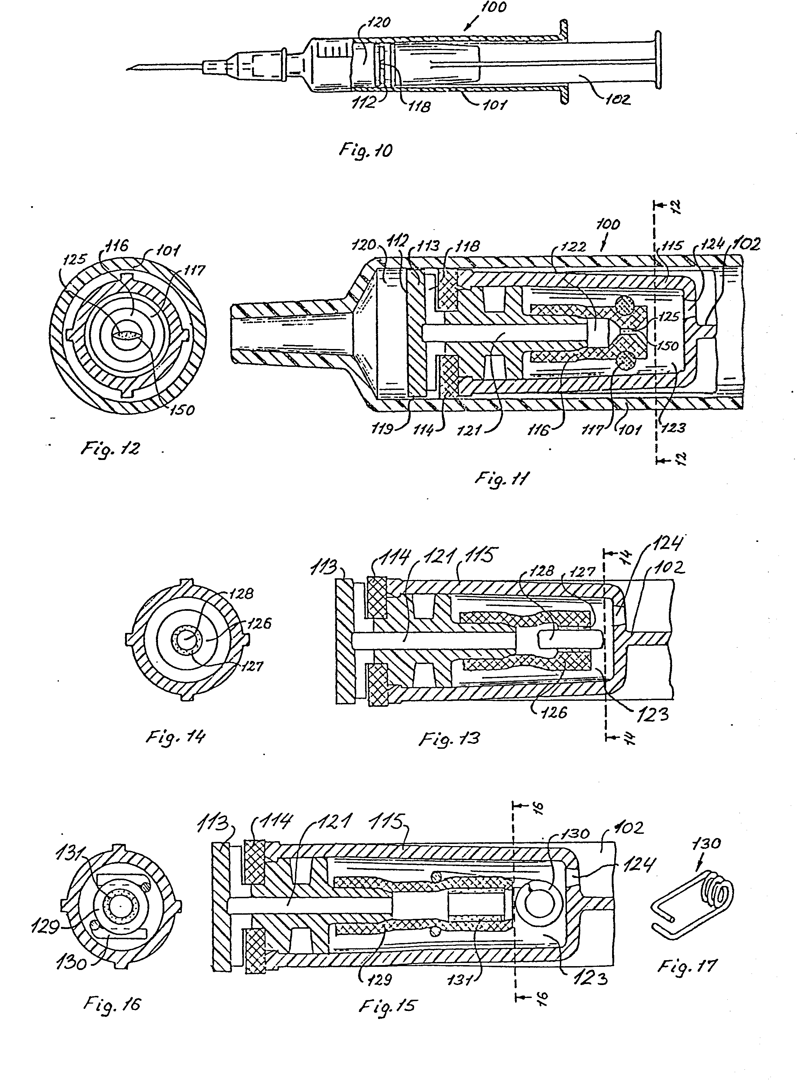

Alternative embodiments of a blood collecting syringe according to the invention will now be described with reference to Figs. 10-17. In Figs. 10-17 the valve means adapted to automatically close the venting passage of the syringe are different from those described above, while the remaining syringe parts are substantially the same as those described above with reference to Figs. 1-9.

Fig. 10 is a side view and partially sectional view of a syringe 100 in which automatically closing valve means which are shown in Figs. 11 and 12, Figs. 13 and 14, and in Figs. 15-17, respectively, may be incorporated. The syringe 100 comprises a piston or plunger 112 of a structure differing substantially from the structure of the piston 12 of the syringe 10 described above. The remaining parts of the syringe 100 correspond substantially to those of the syringe 10 described above, and therefore they will not be further described in the following.

The syringe shown in Fig. 11 comprises a syringe cylinder or barrel 101 in which the piston or plunger 112 is displaceably arranged. The piston 112 comprises a front end element 113 which is shaped substantially like the front end element 19 described above with reference to Figs. 1-9, and which has a sealing ring 114. An annular space 119 is defined between the peripheral outer surface of the piston element 113 and the inner surface of the cylinder 101, and one or more radially extending connecting passages 118 connect the annular space 119 with an inner hollow space or axial bore 121 formed in the piston element 113. Consequently, a blood collecting chamber 120 defined by the piston 112 within the cylinder 101 is in communication with the inner space 121 through the annular space 119 and the connecting passages 118. The piston element 113 comprises a tubular extension, on which automatically functioning valve means are mounted. The valve means comprise a cap member 116 of an elastic material, such as rubber or plastic, mounted around the tubular extension of the piston element 113. A slit or slot 150 is formed or cut in an end wall of the cap member 116, and an elastic ring 117, which may be made from rubber, plastic or another elastic material, is in tight engagement with the outer peripheral surface of the cap member 116 so as to exert compressive forces thereto tending to close the slit or slot 150. However, particles 125, such as crystals, of a water soluble material, for example a mineral salt, are arranged in the slot 150 so as to keep it open and allow gas to pass therethrough as best seen from the cross-sectional view shown in Fig. 12.

When a syringe as that shown in Figs. 11 and 12 is used for collecting an arterial blood sample, and arterial blood flows into the blood collecting chamber 120 under the influence of arterial blood pressure as described above, gas or air may escape from the blood collecting chamber 120 through a venting passage formed by the annular space 119, the connecting passages 118, the axial bore 121, the inner space 122 of the cap member 116, an inner space 123 formed in an inner end portion 115 of a piston rod 102, which is connected to the piston or plunger 112, and one or more openings 124 formed in the inner end portion of the piston rod and connecting the inner space 123 with the atmosphere. When all air or gas has been displaced from the blood collecting chamber 120 by arterial blood, the blood flows into the venting passage defined in the piston and reaches the slot 150. The crystals 125 are then dissolved by the blood, and the slot 150 is now closed under the inward bias of the elastic ring 117.

Figs. 13 and 14 illustrate a further embodiment of the automatically closing valve means. In this embodiment the valve means comprise an elastic tube or hose section 126 made from rubber, plastic, or a similar plastic material. The outer end portion of the hose section 126 has a reduced inner diameter and an increased wall thickness and receives a cylindrical solid core member 128 having an outer diameter exceeding the inner diameter of the surrounding inner surface of the unstrained hose section. Water soluble particles 127, such as crystals, are arranged between the core member 128 and the surrounding part of the hose section 126 so as to define an air passage between the core member 128 and the surrounding hose section 126. Thus, this passage defined by the crystals forms part of the venting passage of the piston. When blood reaches the space defined between the core member 128 and the surrounding hose section 126, the crystals are dissolved and the inner surface of the distended surrounding hose section comes into sealing engagement with the outer surface of the core member 128 so that the venting passage is automatically closed. The water soluble particles 125 and 127 may, for example, be crystals of KJ or KCl or other easily soluble mineral salts.

Figs. 15-17 illustrate a further embodiment of the automatic closing valve means. In this case the valve means comprise a tube or hose section 129 made from rubber, plastic or a similar material. An elastic clamping device 130, which is shown in Fig. 17, engages the outer surface of the free end of the hose section 129 and tends to compress the hose section. A rigid body 131 which is inserted in the bore of the hose section prevents the hose section from collapsing under the bias of the clamping device 130. In the embodiment shown in the drawings the body 131 is in the form of a tube. Alternatively, this body may have any other shape provided that it allows gas to pass through the hose or tube section 129. Thus, the body 131 could be solid, but porous and gas permeable. The body 129 is made from a material which is softened when contacted by blood or an aqueous liquid. Therefore, when blood flows from the blood collecting chamber into the venting passage defined in the piston and reaches the body 131, this body is softened and is then not able to resist the bias of the clamping device 130. Consequently, the hose or tube section 129 is pinched or compressed by the clamping device so that the venting passage is automatically closed.

It should be understood that various changes and modifications of the various embodiments described could be made within the scope of the present invention. However, in all of the embodiments the closing of the valve means controlling the venting passage is actuated when the liquid sample collected comes into contact with a liquid reacting substance whereby the characteristics of this substance are changed in such a manner that this change causes the valve surface parts made from liquid resistant material to come into effectual sealing engagement so as to close the venting passage.

P&V F3563 jB, KEV/IPb, 1983 08 23

CLAIMS

1. A liquid sampler comprising a sample container (11, 101) for receiving a liquid sample in the inner space (17, 120) thereof and having a sample inlet passage (18) and a venting passage (25-31; 118, 119, 121, 122, 150, 124) each communicating with the inner space of the sample container, a liquid reacting substance (22; 127; 131; 150) arranged so as to become exposed to liquid flowing into the venting passage, characterized in that the venting passage (25-31; 118, 119, 121, 122, 150, 124) is controlled by valve means (19, 21; 116, 117; 126, 128; 129-131) defining valve surface parts, which are made from a liquid resistant material and at least partly define the venting passage (25-31; 118, 119, 121, 122, 150, 124), and which are mutually movable from an open position to a closed position in which the venting passage is closed, and in that the liquid reacting substance (22, 125, 127, 131) is adapted to cause said valve surface parts to move from their open to their closed position when said substance has been exposed to and has reacted with liquid.

2. A liquid sampler according to claim 1, characterized in that the liquid reacting substance (22) comprises a substance which expands when contacted by liquid so as to move said valve surface parts into mutually sealing engagement.

3. A liquid sampler according to claim 2, characterized in that the liquid reacting substance comprises a body (22) including fibrous material of a type which swells when contacted by liquid.

4. A liquid sampler according to any of the claims 1-3, characterized in that said valve means comprise a movable valve body (21) and a valve seat (19), the liquid reacting substance (22) being arranged so as to move the valve body into sealing engagement with the valve seat when expanding.

P&V F3563 jB, KEV/IPb, 1983 08 23

5. A liquid sampler according to any of the claims 1-4, characterized in that the liquid reacting substance (22) is arranged within the venting passage downstream of the valve means (19, 21).

6. A liquid sampler according to claim 5, characterized in that the liquid reacting substance (22) is located within an enlarged section (34) of the venting passage, and that the valve body (21) is arranged at the inlet end of the enlarged section, said inlet end forming the valve seat.

7. A liquid sampler according to claim 6, characterized in that the valve body is a disc-like member (21) arranged between the liquid reacting substance (22) and the inlet end of the enlarged section (34).

8. A liquid sampler according to claim 7, characterized in that the disc-like member (36) is hinge-connected so as to be swingable from an open to a closed, valve seat engaging position.

9. A liquid sampler according to claim 1, characterized in that the valve surface parts are biased towards their closed position, and that the liquid reacting substance comprises a material (125, 127, 131), which in its dry condition is rigid and retains the valve surface parts in their open position, and which becomes unable to counteract the bias of the valve surface parts when contacted by liquid, whereby the valve surface parts are moved to their closed position when the liquid reacting substance comes into contact with liquid.

10. A liquid sampler according to claim 9, characterized in that the valve surface parts comprise opposed, spaced surface parts defining a slot (150) therebetween.

11. A liquid sampler according to claim 9, characterized in that the valve surface parts are formed by the inner surface of an elastic hose section (126) and the outer peripheral

P&V F3563 jB, KEV/lPb, 1983 08 23

surface of a cylindrical body (128) arranged within the hose section, the diameter of the cylindrical body exceeding the diameter of the inner surface of the hose section in its non-distended condition, and the rigid liquid reacting substance (127) being arranged between the outer surface of the cylindrical member and the inner surface of the hose section so as to define a passage therebetween.

12. A liquid sampler according to any of the claims 9-11, characterized in that the liquid reacting substance is a liquid soluble material (125, 127).

13. A liquid sampler according to claim 12, characterized in that the liquid soluble material comprises crystals of a mineral salt.

14. A liquid sampler according to claim 13, characterized in that the mineral salt is selected from the group consisting of KCl and KJ.

15. A liquid sampler according to claim 9, characterized in biasing means (130) for biasing the valve surface parts towards their closed position, the liquid reacting substance (131) being liquid softenable, whereby the valve surface parts are moved to their closed position by the biasing means when the liquid reacting substance comes into contact with liquid.

16. A liquid sampler according to claim 9, characterized in that the valve surface parts comprise inner surface parts of a compressible tubular member (116, 126, 129).

17. A liquid sampler according to claim 16, characterized in that the biasing means is an elastic clamping device (130) engaging with the outer surface of the tubular member.

18. A liquid sampler according to claim 16 or 17, characterized in that the liquid reacting substance is in the form of a gas permeable rigid body (131), which becomes softened and compressible when contacted by liquid.

P&V F3563 jB, KEV/IPb, 1983 08 23

19. A liquid sampler according to any of the claims 1-18, characterized in that the inner space (17, 120) of the sample container (11, 101) is variable.

20. A liquid sampler according to claim 19, characterized in that the sample container is in the form of a syringe cylinder (11, 101) and a piston (12, 112) movably arranged therein.

21. A liquid sampler according to claim 20, characterized in that the venting passage communicates with the inner space (17, 120) of the syringe cylinder through a slot (24, 119) defined between the inner peripheral wall of the cylinder and the outer peripheral wall of the piston.

22. A liquid sampler according to claim 20 or 21, characterized in that the venting passage is at least partly defined in the piston.

P&V F3563 jB, KEV/IPb, 1983 08 23

Fig. 1

Fig. 2

Fig. 3

Fig. 4

Fig. 5

Fig. 6

Fig. 7A

Fig. 7B

Fig. 7C

Fig. 8A

Fig. 8B

Fig. 8C

Fig. 9A

Fig. 9B

Fig. 9C

4/4

100
120
112 118 101 102

Fig. 10

125 116 101 117
150

Fig. 12

120 112 113 118 122 100 115 124 102
119 114 121 116 117 101 123

Fig. 11

113 114 121 115 128 127 124 102
128
126
127
126 123

Fig. 14

Fig. 13

113 114 121 115 130 102
131
129
130
129 131 123
124

Fig. 16

Fig. 15

130

Fig. 17